# EUROPEAN PATENT SPECIFICATION

(11) **EP 3 153 197 B1**
(45) Date of publication and mention of the grant of the patent: **03.08.2022**
(21) Application number: 16195292.4
(22) Date of filing: 05.12.2012
(51) Int. Cl.: A61M 5/32, A61M 5/20, A61M 5/24, A61M 5/34

(54) **SYRINGE CARRIER**
SPRITZENTRÄGER
SUPPORT DE SERINGUE

(30) Priority: 08.12.2011 EP 11192585
(43) Date of publication of application: 12.04.2017
(62) Divisional of application: 12794996.4
(73) Proprietor: Sanofi-Aventis Deutschland GmbH, 65929 Frankfurt am Main (DE)
(72) Inventor: HOURMAND, Yannick, Haslingfield, Cambridgeshire CB23 1ND (GB); JENNINGS, Douglas Ivan, Royston, Hertfordshire SG8 7XU (GB); EKMAN, Matthew, Macclesfield, Cheshire SK10 1RD (GB)
(74) Representative: Schmidt, Christian

(56) References cited:
- EP-A1- 2 727 617
- WO-A1-01/08727
- WO-A1-2010/136078
- GB-A- 407 109
- GB-A- 2 471 473
- US-A- 5 865 805
- US-A1- 2006 161 114

## Description

### Technical Field

The invention relates to syringe carrier.

### Background of the Invention

In a conventional medicament delivery device (e.g., an autoinjector), a pre-filled syringe is housed in a carrier which is axially movable to achieve needle penetration in an injection site and, optionally, needle withdrawal. A conventional carrier provides shoulders that are adapted to engage a neck on the syringe and prevent the syringe from disengaging the carrier. Because syringes are generally supplied with rigid needle shields covering the needle and those needle shields have a diameter greater than a diameter between the shoulders, a separate assembly step is required - inserting the syringe in the carrier and then attaching the rigid needle shield to the needle. Accordingly, there is a need for a syringe carrier which does not require this separate assembly step.

WO 2001/08727 A1 discloses an adapter including a syringe carrier adapted to seat at least a portion of the syringe. The syringe carrier includes at least one rearward facing abutment member to abut at least one forward facing surface of a syringe. The syringe carrier includes an opening therein to allow a drive member of an injector to Communicate forward force to the plunger through abutment without connective engagement between the drive member and the plunger. The adapter further includes a releasable mounting mechanism positioned to the rear of the syringe carrier to mount the adapter in a desired position relative to the front wall of the injector. An adapter includes a first section and a second section that are rotatable relative to each other about a hinge axis generally perpendicular to a longitudinal axis of the adapter. An adapter includes a first section and a second section that are generally the same in construction, the first section and the section being connectable to form a syringe carrier to seat at least a portion of the syringe.

US 5 865 805 A discloses a power injector for injecting fluid from a replaceable syringe into the body of an animal. The syringe includes a syringe adapter interface for accepting side mountable syringes having pushrod extensions for actuating the syringe plungers from a point behind the syringe body. The injector is provided with an operator-removable and interchangeable interface in the form of a syringe mounting head. The interface includes a holder that has two halves, one fixed and one moveable, that open and close in a clam-shell fashion to respectively load or unload a syringe and to operate the syringe by driving the plunger thereof with a power driven ram that extends form the injector housing. The end of the injector ram, which is configured to normally couple to a button or key on the back of the plunger of a front or rear loadable syringe, is provided with an adapter that couples to the ram and to the large disk shaped end of a side loadable syringe pushrod. The adapter has opposed spring biased gripping fingers projecting forward of a coupling plate on the adapter in order to clip the pushrod end to the adapter. The fingers are configured and spaced relative to the coupling plate to engage the pushrod end either upon axial advancement against it or when the syringe is translated sideways into the holder.

EP 2 727 617 A1, published after the priority date of the present application, discloses an autoinjector comprising a case, a door hingedly coupled to the case and having an open position and a closed position, a plunger slidably disposed in the case, and at least one drive spring applying a biasing force on the plunger relative to the case, wherein the door is operably coupled to the plunger, and wherein rotation of the door from the closed position to the open position moves the plunger from a distal position in the case to a proximal position in the case and compresses the at least one drive spring.

WO 2010/136078 A1 discloses an injection device for administering a product, comprising a distal end and a needle which is located inside the injection device in the initial position and can be moved into a puncturing position in which the needle projects from the distal end of the injection device. Said injection device further comprises: a) an opening region which is located in the distal direction of the needle in the initial position of the needle and has a certain cross-section; b) a reduction piece which can be moved into the opening region such that the cross-section of the opening region is reduced.

GB 2471473 A discloses a device for unsheathing the needle of a syringe has a cylindrical housing on which is mounted a driver sleeve that can be moved along the outside of the housing. The driver is coupled to resilient fingers which form a collar on the inside of the housing. A syringe with a sheath covering its needle can be inserted into the housing through an opening to engage with a syringe carrier. To remove the sheath, the driver is pulled rearwardly until it meets a step in the housing, causing the fingers to ride over the sheath and engage a gap between the top of the sheath and the syringe barrel. The driver is then pushed forwards, back to its original position, causing the fingers to push the sheath off the needle.

GB 407,109 A discloses a hypodermic syringe having a part of the body which is hingedly mounted, and longitudinally slidable on the other part, and is rigidly connected with a member which clamps the needle in position so that, when a closure cap is moved forward, the parts unite to form the syringe body while the clamping member is accurately positioned relative to the syringe head. The part is pivoted at to a cylindrical slide held to the cap by a ring. The member is wedge-shaped and enters a recess in the syringe head, wing pieces being slid -forward beneath undercut edges of the head when the cap is tightened by a bayonet joint comprising a pin engaging a groove in the syringe. The slide is cut away at its front end and at the rear has a beading, overlapped by the margin of the ring which has a recess through which the bead can enter. The pressure rod carries a claw recessed at to receive the plunger knob of a liquid container.

### Summary of the Invention

It is an object of the present invention to provide an improved syringe carrier and a method for assembling a syringe comprising said syringe carrier.

According to the invention, the object is achieved by a syringe carrier with the features of claim 1 and a method with the features of claim 13.

In an exemplary embodiment, a syringe carrier according to the present invention comprises a body adapted to receive a barrel of a syringe. The body includes two sections having distal ends with shoulder sections adapted to engage a circumferential gap between the barrel of the syringe and a needle shield covering a needle of the syringe.

In an illustrative embodiment, the sections are resiliently coupled to a collar on a proximal end of the body. The shoulder sections deflect when engaged by the needle shield and return to a non-deflected position when disengaged by the needle shield to engage the circumferential gap between the barrel of the syringe and the needle shield.

In an illustrative embodiment, the sections are resiliently coupled to a collar on a distal end of the body. The sections deflect when engaged by the needle shield and return to a non-deflected position when disengaged by the needle shield to engage a finger flange of the syringe. The body includes resilient arms having additional shoulder sections adapted to engage the circumferential gap between the barrel of the syringe and a needle shield covering a needle of the syringe. The arms deflect when engaged by the needle shield and return to a non-deflected position when disengaged by the needle shield to engage the circumferential gap between the barrel of the syringe and a needle shield.

In an exemplary embodiment, the sections are coupled via at least one hinge and are movable between an open position and a closed position. A first section includes a pin adapted to engage a hole on a second section to secure the sections in the closed position.

In an exemplary embodiment, the shoulder sections include facing surfaces, wherein the pin and hole are included in the facing surfaces, wherein, when the sections are in a closed position, the facing surfaces abut each other so that the shoulder sections form circular shoulders to distally engage the circumferential gap between the barrel and the needle shield.

In an exemplary embodiment, when the sections are in a closed position, the facing surfaces abut each other so that the shoulder sections form circular shoulders adapted to proximally abut a finger flange on the syringe.

In an exemplary embodiment, the pin is adapted to engage the hole by friction or by a snap-fit.

In an illustrative embodiment, the sections are coupled via at least one clip and are movable between an open position and a closed position. The at least one clip includes a hook on a first section adapted to engage an eye on a second section to secure the sections in the closed position.

In an illustrative embodiment, the sections include doors hingedly coupled to the body and additional shoulder sections are formed on distal ends of the doors.

In an exemplary embodiment, the shoulder sections include proximally-facing contoured surfaces to accommodate a proximal portion of a neck of the syringe and distally-facing planar surfaces to abut the needle shield.

In an exemplary embodiment, the body includes one or more viewing windows.

In an exemplary embodiment, the body includes a retainer element adapted to provide an abutment surface to prevent the syringe from disengaging the syringe carrier in a proximal direction.

Further scope of applicability of the present invention will become apparent from the detailed description given hereinafter. However, it should be understood that the detailed description and specific examples, while indicating preferred embodiments of the invention, are given by way of illustration only, since various changes and modifications within the scope of the invention, as defined in the appended claims, will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a top view of an illustrative embodiment of a syringe carrier,
- Figure 2: is a lateral view of the syringe carrier of figure 1,
- Figure 3: is a longitudinal section of the syringe carrier of figure 1 in the section plane A-A,
- Figure 4: is a perspective view of the syringe carrier of figure 1,
- Figure 5: is a top view of another illustrative embodiment of a syringe carrier,
- Figure 6: is a lateral view of the syringe carrier of figure 5,
- Figure 7: is a longitudinal section of the syringe carrier of figure 5 in the section plane A-A,
- Figure 8: is a perspective view of the syringe carrier of figure 5,
- Figure 9: is a top view of yet another exemplary embodiment of a syringe carrier according to the present invention,
- Figure 10: is a lateral view of the syringe carrier of figure 9,
- Figure 11: is a longitudinal section of the syringe carrier of figure 9 in the section plane A-A,
- Figure 12: is a perspective view of the syringe carrier of figure 9,
- Figure 13: is another perspective view of the syringe carrier of figure 9 with a syringe inserted,
- Figure 14: is a top view of yet another illustrative embodiment of a syringe carrier,
- Figure 15: is a lateral view of the syringe carrier of figure 14,
- Figure 16: is a longitudinal section of the syringe carrier of figure 14 in the section plane A-A,
- Figure 17: is a perspective view of the syringe carrier of figure 14,
- Figure 18: is another perspective view of the syringe carrier of figure 14 with a syringe inserted,
- Figure 19: is a top view of yet another illustrative embodiment of a syringe carrier,
- Figure 20: is a lateral view of the syringe carrier of figure 19,
- Figure 21: is a longitudinal section of the syringe carrier of figure 19 in the section plane A-A,
- Figure 22: is a perspective view of the syringe carrier of figure 19,
- Figure 23: is another perspective view of the syringe carrier of figure 19 with a syringe inserted,
- Figure 24: is a top view of yet another illustrative embodiment of a syringe carrier,
- Figure 25: is a lateral view of the syringe carrier of figure 24,
- Figure 26: is a longitudinal section of the syringe carrier of figure 24 in the section plane A-A,
- Figure 27: is a perspective view of the syringe carrier of figure 24,
- Figure 28: is another perspective view of the syringe carrier of figure 24 with a syringe inserted,
- Figure 29: is a top view of yet another illustrative embodiment of a syringe carrier,
- Figure 30: is a lateral view of the syringe carrier of figure 29,
- Figure 31: is a longitudinal section of the syringe carrier of figure 29 in the section plane A-A,
- Figure 32: is a perspective view of the syringe carrier of figure 29,
- Figure 33: is another perspective view of the syringe carrier of figure 29 with a syringe inserted,
- Figure 34: is a top view of yet another illustrative embodiment of a syringe carrier,
- Figure 35: is a lateral view of the syringe carrier of figure 34,
- Figure 36: is a longitudinal section of the syringe carrier of figure 34 in the section plane A-A,
- Figure 37: is a perspective view of the syringe carrier of figure 34,
- Figure 38: is another perspective view of the syringe carrier of figure 34 with a syringe inserted,
- Figure 39: is a top view of yet another illustrative embodiment of a syringe carrier,
- Figure 40: is a lateral view of the syringe carrier of figure 39,
- Figure 41: is a longitudinal section of the syringe carrier of figure 39 in the section plane B-B,
- Figure 42: is a perspective view of the syringe carrier of figure 39,
- Figure 43: is another perspective view of the syringe carrier of figure 39 with a syringe inserted,
- Figure 44: is a top view of yet another illustrative embodiment of a syringe carrier,
- Figure 45: is a lateral view of the syringe carrier of figure 44,
- Figure 46: is a longitudinal section of the syringe carrier of figure 44 in the section plane B-B,
- Figure 47: is a perspective view of the syringe carrier of figure 44, and
- Figure 48: is another perspective view of the syringe carrier of figure 44 with a syringe inserted.

Corresponding parts are marked with the same reference symbols in all figures.

### Detailed Description

Generally, and applicable to all exemplary embodiments of the present invention, the syringe 2 comprises a barrel 2.1 and a neck 2.2 which has a smaller diameter than the barrel 2.1. A needle 3 is mounted to the neck 2.2 and a rigid needle shield (RNS) 4 is removably arranged on the needle 3. When coupled to the needle 3, a portion of the RNS may cover a portion of the neck 2.2, leaving a circumferential gap between the barrel 2.1 and the RNS 4. The RNS 4 has a diameter substantially equal to the diameter of the barrel 2.1.

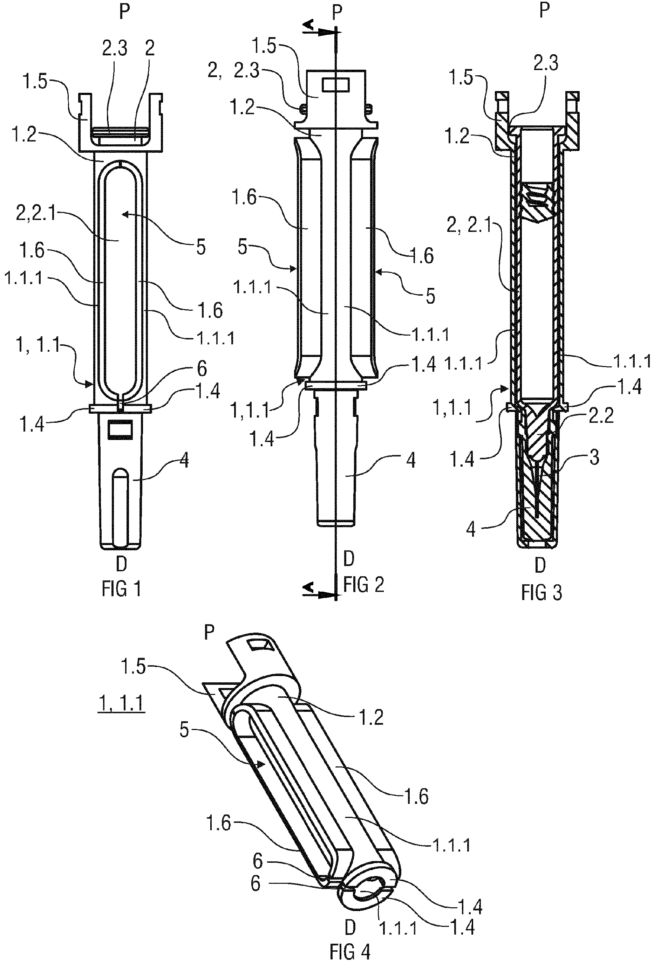

Figures 1-4 show a first illustrative embodiment of a syringe carrier 1. Figure 1 is a top view of the syringe carrier 1 for supporting a syringe 2. Figure 2 is a lateral view of the syringe carrier of figure 1. Figure 3 is a longitudinal section of the syringe carrier of figure 1 in the section plane A-A. Figure 4 is a perspective view of the syringe carrier of figure 1 without the syringe 2.

As shown in Figures 1-4, the syringe carrier 1 comprises an elongate body 1.1 arranged to receive the barrel 2.1. In this exemplary embodiment, the body 1.1 has a cylindrical shape with an internal diameter corresponding to the diameter of the barrel 2.1. The body 1.1 comprises a collar 1.2 at a proximal end dimensioned to allow axial insertion of the syringe 2 into the syringe carrier 1 in a distal direction D. Resilient sections 1.1.1 extend distally from the collar 1.2. Distal ends of the sections 1.1.1 include shoulder sections 1.4 shaped as portions of a circle arranged in a transverse plane with respect to a longitudinal axis of the carrier 1. The shoulder sections include facing surfaces 6. When the sections 1.1.1 are in a non-deflected position, the facing surfaces 6 may abut each other, and the shoulder sections 1.4 form a circular shoulder (because the facing surfaces 6 abut each other) adapted to engage the circumferential gap between the barrel 2.1 and the RNS 4.

The syringe 2, with RNS 4 attached to the needle 3, may be loaded into the syringe carrier 1 by sliding the syringe 2 in the distal direction D into the syringe carrier 2. When the RNS 4 abuts the shoulder sections 1.4, additional axial force may be applied to cause the arms 1.3 to deflect radially. When the RNS 4 has bypassed the shoulder sections 1.4, the sections 1.1.1 may return to the non-deflected position, and the shoulder sections 1.4 may engage the circumferential gap between the barrel 2.1 and the RNS 4 and prevent the syringe 2 from moving in the distal direction D relative to the syringe carrier 1.

In an exemplary embodiment, the proximal end 1.5 of the body 1.1 may be arranged to receive a finger flange 2.3 of the syringe 2.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, viewing windows 5 may be arranged in the body 1.1 for allowing visual access to the barrel 2.1 of the syringe 2 when the syringe 2 is in the syringe carrier 2. In an exemplary embodiment, the windows 5 are formed when cut-outs in the arms 1.3 are substantially contiguous when the arms 1.3 are in the non-deflected position (as shown in Figure 1). A projection 1.6 may be formed around each cut-out, and when the sections 1.1.1 are in the non-deflected position, the projections 1.6 may form an outline for the window 5. In another exemplary embodiment, the windows 5 may be formed in the sections 1.1.1.

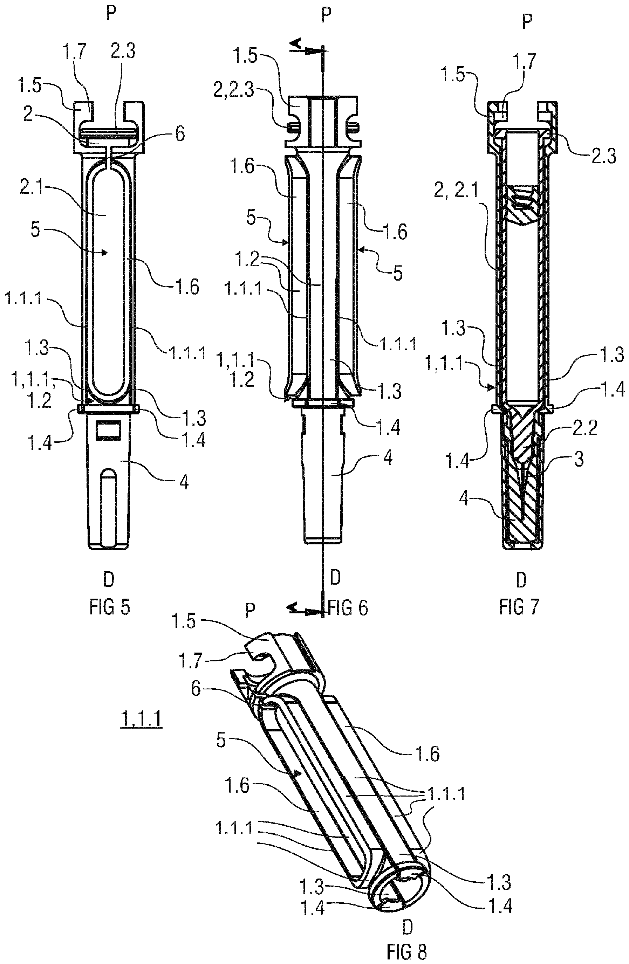

Figures 5-8 show a second illustrative embodiment of a syringe carrier 1. Figure 6 is a lateral view of the syringe carrier 1 of figure 5. Figure 7 is a longitudinal section of the syringe carrier 1 of figure 5 in the section plane A-A. Figure 8 is a perspective view of the syringe carrier of figure 5 without the syringe 2.

As shown in Figures 5-8, the syringe carrier 1 comprises an elongate body 1.1 arranged to receive the barrel 2.1. In this exemplary embodiment, the body 1.1 is comprised of two resilient sections 1.1.1 which, when together, have a cylindrical shape with an internal diameter corresponding to the diameter of the barrel 2.1. Distal ends of the sections 1.1.1 of the body 1.1 comprise part of a collar 1.2 dimensioned to allow axial insertion of the syringe 2 into the syringe carrier 1. Resilient arms 1.3 are formed in the body 1.1. Distal ends of the arms 1.3 include shoulder sections 1.4 shaped as portions of a circle arranged in a transverse plane with respect to a longitudinal axis of the carrier 1. The shoulder sections include facing surfaces 6. When the arms 1.3 are in a non-deflected position, the facing surfaces 6 may abut the distal ends of the sections 1.1.1 of the body 1.1 to form a circular shoulder adapted to engage the circumferential gap between the barrel 2.1 and the RNS 4.

The syringe 2, with RNS 4 attached to the needle 3, may be loaded into the syringe carrier 1 by sliding the syringe 2 in the distal direction D into the syringe carrier 2. When the RNS 4 abuts proximal ends of the sections 1.1.1, the sections 1.1.1 may deflect radially. When the RNS 4 has bypassed the proximal ends of the section 1.1.1, the sections 1.1.1 may return to the non-deflected position. When the RNS 4 abuts the shoulder sections 1.4, the arms 1.3 may deflect until the RNS 4 bypasses the shoulder sections 1.4. Then, the arms 1.3 may return to the non-deflected position, and the shoulder sections 1.4 and the collar 1.2 may engage the circumferential gap between the barrel 2.1 and the RNS 4 and prevent the syringe 2 from moving in the distal direction D relative to the syringe carrier 1.

In an exemplary embodiment, the proximal end 1.5 of the body 1.1 may be arranged to receive a finger flange 2.3 of the syringe 2. The proximal end 1.5 may also include a retainer element 1.7 which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, viewing windows 5 may be arranged in the body 1.1 for allowing visual access to the barrel 2.1 of the syringe 2 when the syringe 2 is in the syringe carrier 2. In an exemplary embodiment, the windows 5 are formed when cut-outs in the sections 1.1.1 are substantially contiguous when the sections 1.1.1 are in the non-deflected position (as shown in Figure 5). A projection 1.6 may be formed around each cut-out, and when the sections 1.1.1 are in the non-deflected position, the projections 1.6 may form an outline for the window 5.

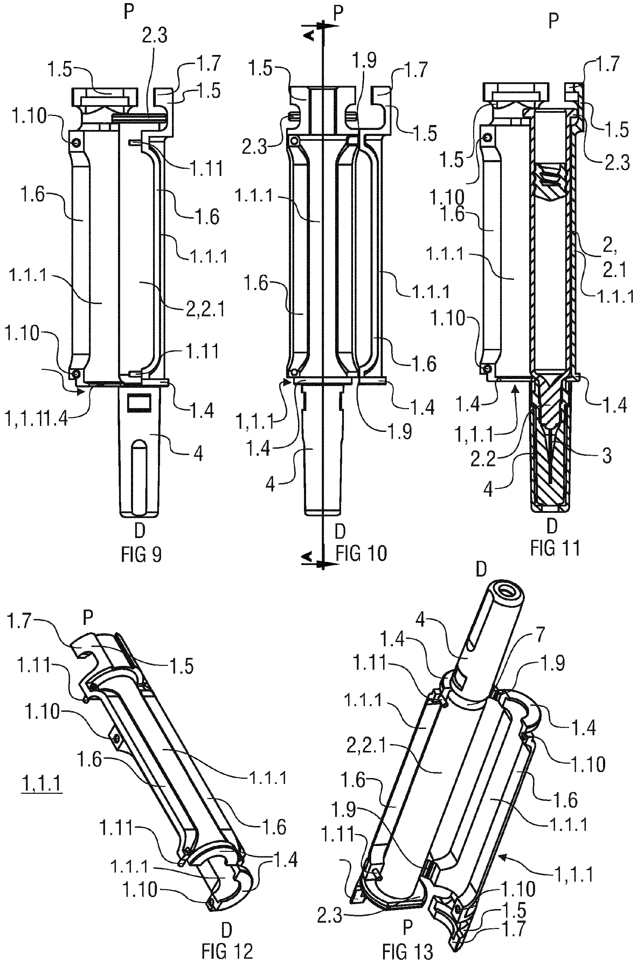

Figures 9-13 show a third exemplary embodiment of a syringe carrier 1 according to the present invention. Figure 9 is a top view of a third embodiment of a syringe carrier 1 for supporting a syringe 2. Figure 10 is a lateral view of the syringe carrier 1 of figure 9. Figure 11 is a longitudinal section of the syringe carrier 1 of figure 9 in the section plane A-A. Figure 12 is a perspective view of the syringe carrier of figure 9 without the syringe 2. Figure 13 is another perspective view of the syringe carrier of figure 9.

As shown in Figures 9-13, the syringe carrier 1 comprises an elongate body 1.1 arranged to receive the barrel 2.1. In this exemplary embodiment, the body 1.1 is comprised of two sections 1.1.1 which, when together, have a cylindrical shape with an internal diameter corresponding to the diameter of the barrel 2.1. The sections 1.1.1 may be coupled by a side hinge which allows the section 1.1.1 to rotate relative to each other sufficient to receive the syringe 2. Proximal and distal ends of the sections 1.1.1 include shoulder sections 1.4 shaped as portions of a circle arranged in a transverse plane with respect to a longitudinal axis of the carrier 1. The shoulder sections include facing surfaces 6. When the sections 1.1.1 are in a closed position, the facing surfaces 6 may abut each other so that the shoulder sections 1.4 form circular shoulders adapted to proximally abut a finger flange 2.3 on the syringe 2 and to distally engage the circumferential gap between the barrel 2.1 and the RNS 4. The facing surfaces 6 of one section 1.1.1 may include holes 1.10 and the facing surfaces 6 of the other section 1.1.1 may include pins 1.11 adapted to engage (e.g., frictionally, snap-fit, etc.) the holes 1.10 to secure the sections 1.1.1 in the closed position.

The syringe 2, with RNS 4 attached to the needle 3, may be loaded into the syringe carrier 1 by opening the sections 1.1.1 about the hinge and placing the syringe 2 in the syringe carrier 2. When the sections 1.1.1 are closed, the pins 1.11 engage the holes 1.10, and the proximal shoulder sections 1.4 form circular shoulders adapted to proximally abut a finger flange 2.3 on the syringe 2 and the distal shoulder section s1.4 to distally engage the circumferential gap between the barrel 2.1 and the RNS 4. Thus, the syringe 2 is prevented from moving axially relative to the syringe carrier 1.

In an exemplary embodiment, the proximal end 1.5 may include a retainer element 1.7 which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, viewing windows 5 may be arranged in the body 1.1 for allowing visual access to the barrel 2.1 of the syringe 2 when the syringe 2 is in the syringe carrier 2. In an exemplary embodiment, the windows 5 are formed when cut-outs in the sections 1.1.1 are substantially contiguous when the sections 1.1.1 are in the closed position. A projection 1.6 may be formed around each cut-out, and when the sections 1.1.1 are in the non-deflected position, the projections 1.6 may form an outline for the window 5.

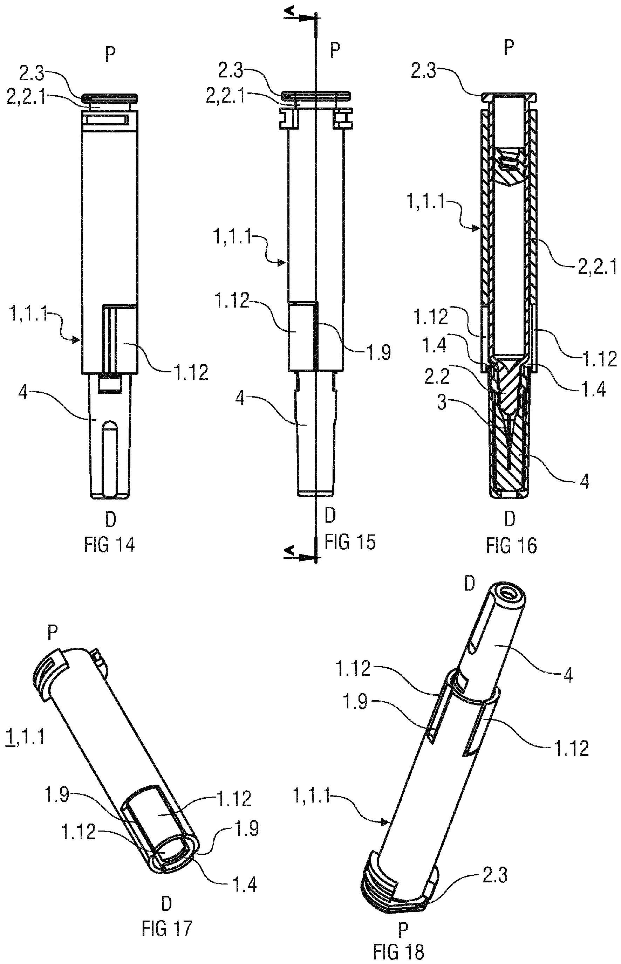

Figures 14-18 show a fourth illustrative embodiment of a syringe carrier 1. Figure 14 is a top view of a fourth embodiment of a syringe carrier 1 for supporting a syringe 2. Figure 15 is a lateral view of the syringe carrier 1 of figure 14. Figure 16 is a longitudinal section of the syringe carrier 1 of figure 14 in the section plane A-A. Figure 17 is a perspective view of the syringe carrier of figure 14 without the syringe 2. Figure 18 is another perspective view of the syringe carrier of figure 14.

As shown in Figures 14-18, the syringe carrier 1 comprises an elongate body 1.1 arranged to receive the barrel 2.1. In this exemplary embodiment, the body 1.1 has a cylindrical shape with an internal diameter corresponding to the diameter of the barrel 2.1. A distal end of the body 1.1 includes a shoulder sections 1.4 shaped as a portion of a circle arranged in a transverse plane with respect to a longitudinal axis of the carrier 1, and at least one door 1.12 hingedly coupled to the body 1.1 and including a shoulder section 1.4. A hinge 1.9 coupling the door 1.12 to the body 1.1 may be provided on an axis parallel to the longitudinal axis of the syringe carrier 1 or on an axis transverse to the longitudinal axis of the syringe carrier 1. The shoulder section 1.4 includes facing surfaces 6 which abut facing surfaces 6 of the door 1.12 when the door 1.12 is in a closed position (as shown in Figure 14). When the door 1.12 is in the closed position, the facing surfaces 6 may abut each other so that the shoulder sections 1.4 on the body 1.1 and the door 1.12 to form a circular shoulder adapted to engage the circumferential gap between the barrel 2.1 and the RNS 4. The facing surfaces 6 of the door 1.12 may include holes 1.10 and the facing surfaces 6 of the body 1.1 may include pins 1.11 (or vice-versa) adapted to engage (e.g., frictionally, snap-fit, etc.) the holes 1.10 to secure the door 1.12 in the closed position.

The syringe 2, with RNS 4 attached to the needle 3, may be loaded into the syringe carrier 1 by opening the door 1.12 and sliding the syringe 2 into the syringe carrier 1. When the circumferential gap between the barrel 2.1 and the RNS 4 engages the shoulder section 1.4 on the body 1.1, the door 1.12 may be closed to engage the gap and prevent the syringe 2 from moving axially relative to the syringe carrier 1.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, viewing windows (not shown) may be arranged in the body 1.1 for allowing visual access to the barrel 2.1 of the syringe 2 when the syringe 2 is in the syringe carrier 2. In an exemplary embodiment, the windows are formed as cut-outs.

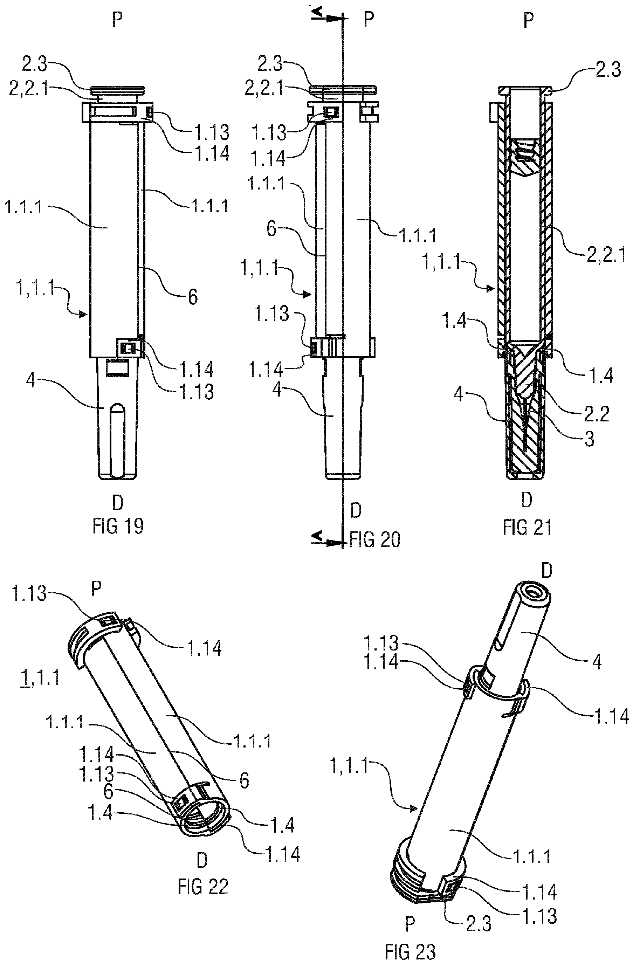

Figures 19-23 show a fifth illustrative embodiment of a syringe carrier 1. Figure 19 is a top view of a fifth embodiment of a syringe carrier 1 for supporting a syringe 2. Figure 20 is a lateral view of the syringe carrier 1 of figure 19. Figure 21 is a longitudinal section of the syringe carrier 1 of figure 19 in the section plane A-A. Figure 22 is a perspective view of the syringe carrier of figure 19 without the syringe 2. Figure 23 is another perspective view of the syringe carrier of figure 19.

As shown in Figures 19-23, the syringe carrier 1 comprises an elongate body 1.1 arranged to receive the barrel 2.1. In this exemplary embodiment, the body 1.1 is comprised of two sections 1.1.1 which, when together, have a cylindrical shape with an internal diameter corresponding to the diameter of the barrel 2.1. The sections 1.1.1 may be coupled together by clips. In an exemplary embodiment, a clip may comprise a eye 1.14 on a first section adapted to engage a hook 1.13 on a second section. The eye 1.14 may have a cross-section substantially equal to the cross-section of the hook 1.13 such that the eye 1.14 and hook 1.13 engage in a snap-fit. Distal ends of the sections 1.1.1 include shoulder sections 1.4 shaped as portions of a circle arranged in a transverse plane with respect to a longitudinal axis of the carrier 1. The shoulder sections include facing surfaces 6. When the sections 1.1.1 are in a closed position, the facing surfaces 6 may abut each other so that the shoulder sections 1.4 form circular shoulders adapted engage the circumferential gap between the barrel 2.1 and the RNS 4. Those of skill in the art will understand that the sections 1.1.1 may be hingedly connected.

The syringe 2, with RNS 4 attached to the needle 3, may be loaded into the syringe carrier 1 by opening the sections 1.1.1 and placing the syringe 2 in the syringe carrier 2. When the sections 1.1.1 are closed, the eyes 1.14 engage the hooks 1.13 and the shoulder sections 1.4 engage the circumferential gap between the barrel 2.1 and the RNS 4. Thus, the syringe 2 is prevented from moving axially relative to the syringe carrier 1.

In an exemplary embodiment, the proximal end may include a retainer element which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, viewing windows may be arranged in the body 1.1 for allowing visual access to the barrel 2.1 of the syringe 2 when the syringe 2 is in the syringe carrier 2.

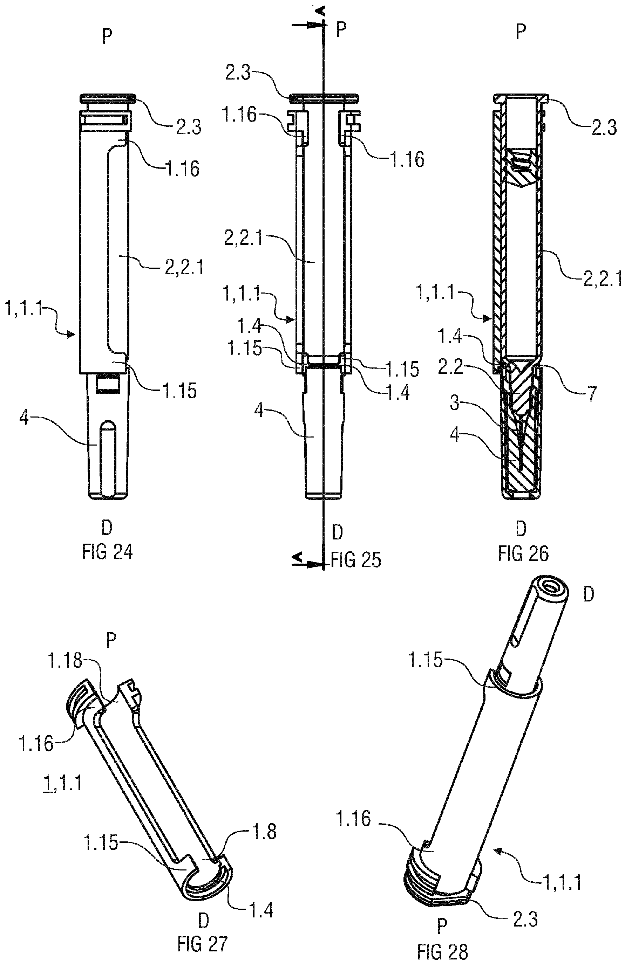

Figures 24-28 show a sixth illustrative embodiment of a syringe carrier 1. Figure 24 is a top view of a sixth embodiment of a syringe carrier 1 for supporting a syringe 2. Figure 25 is a lateral view of the syringe carrier 1 of figure 24. Figure 26 is a longitudinal section of the syringe carrier 1 of figure 24 in the section plane A-A. Figure 27 is a perspective view of the syringe carrier of figure 24 without the syringe 2. Figure 28 is another perspective view of the syringe carrier of figure 24.

As shown in Figures 24-28, the syringe carrier 1 comprises an elongate body 1.1 arranged to receive the barrel 2.1. In this exemplary embodiment, the body 1.1 has a partially cylindrical shape with an internal diameter corresponding to the diameter of the barrel 2.1. The body 1.1 may include a longitudinal slot (e.g., a cut-out) which is adapted to snap over the barrel 2.1 of the syringe 2. Proximal and distal ends of the body 1.1 include clamps 1.15, 1.16 which are adapted to retain the syringe 2 when in the syringe carrier 1. The distal end of the body 1 further includes shoulder sections 1.4 shaped as a portion of a circle arranged in a transverse plane with respect to a longitudinal axis of the carrier 1. The shoulder sections 14 form circular shoulders adapted to engage the circumferential gap between the barrel 2.1 and the RNS 4.

The syringe 2, with RNS 4 attached to the needle 3, may be loaded into the syringe carrier 1 by pressing the barrel 2.1 against the clamps 1.15, 1.16, causing the clamps 1.15, 1.16 to deflect and widen the longitudinal slot in the body 1.1. When the barrel 2.1 bypasses the clamps 1.15, 1.16, the clamps 1.15, 1.16 return to their non-deflected position and retain the syringe 2 in the syringe carrier 1. The shoulder sections 1.4 engage the circumferential gap between the barrel 2.1 and the RNS 4. Thus, the syringe 2 is prevented from moving axially relative to the syringe carrier 1.

In an exemplary embodiment, the proximal end may include a retainer element which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, a viewing window may be arranged in the body 1.1 for allowing visual access to the barrel 2.1 of the syringe 2 when the syringe 2 is in the syringe carrier 2.

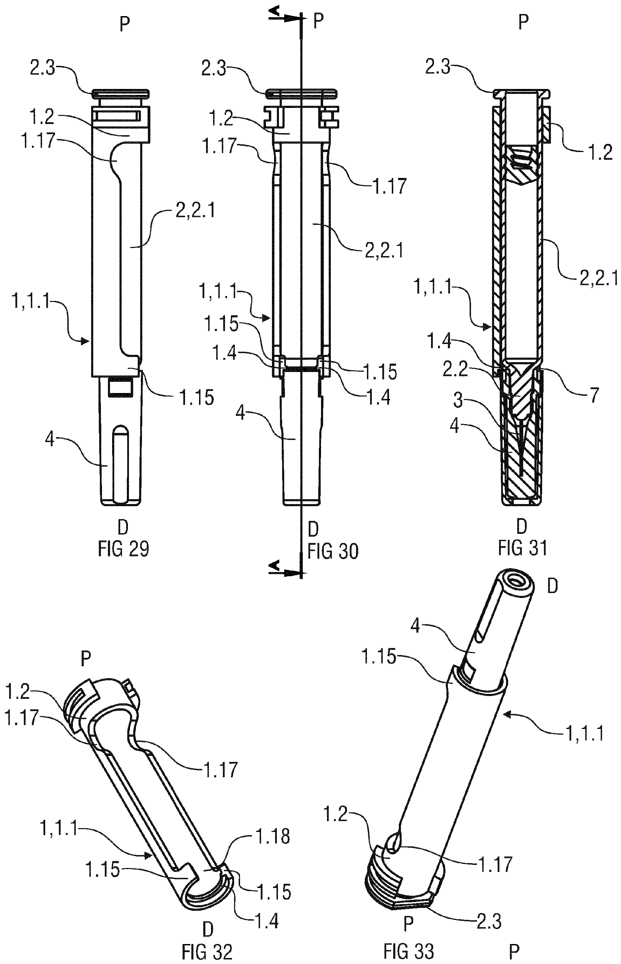

Figures 29-33 show a seventh illustrative embodiment of a syringe carrier 1. Figure 29 is a top view of a seventh embodiment of a syringe carrier 1 for supporting a syringe 2. Figure 30 is a lateral view of the syringe carrier 1 of figure 29. Figure 31 is a longitudinal section of the syringe carrier 1 of figure 29 in the section plane A-A. Figure 32 is a perspective view of the syringe carrier of figure 29 without the syringe 2. Figure 33 is another perspective view of the syringe carrier of figure 29.

As shown in Figures 29-33, the syringe carrier 1 comprises an elongate body 1.1 arranged to receive the barrel 2.1. In this exemplary embodiment, the body 1.1 has a partially cylindrical shape with an internal diameter corresponding to the diameter of the barrel 2.1. The body 1.1 includes a collar 1.2 at its proximal end and may include a longitudinal slot (e.g., a cut-out) formed in the body 1.1 distally of the collar 1.2 which is adapted to snap over the barrel 2.1 of the syringe 2. A pair of groove hinges 1.17 may be formed in the body 1.1 adjacent a proximal end of the slot. The distal end of the body 1 includes shoulder sections 1.4 shaped as a portion of a circle arranged in a transverse plane with respect to a longitudinal axis of the carrier 1. The shoulder sections 14 form circular shoulders adapted to engage the circumferential gap between the barrel 2.1 and the RNS 4.

The syringe 2, with RNS 4 attached to the needle 3, may be loaded into the syringe carrier 1 by sliding the syringe 2 through the collar 1.2 in the distal direction D. When the RNS 4 abuts the shoulder sections 1.4, the body 1.1 may radially deflect (e.g., rotate) about the groove hinges 1.17. When the RNS 4 bypasses the shoulder sections 1.4, the body 1.1 may return to its non-deflected position and retain the syringe 2 in the syringe carrier 1. The shoulder sections 1.4 engage the circumferential gap between the barrel 2.1 and the RNS 4. Thus, the syringe 2 is prevented from moving axially relative to the syringe carrier 1.

In an exemplary embodiment, the proximal end may include a retainer element which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, a viewing window may be arranged in the body 1.1 for allowing visual access to the barrel 2.1 of the syringe 2 when the syringe 2 is in the syringe carrier 2.

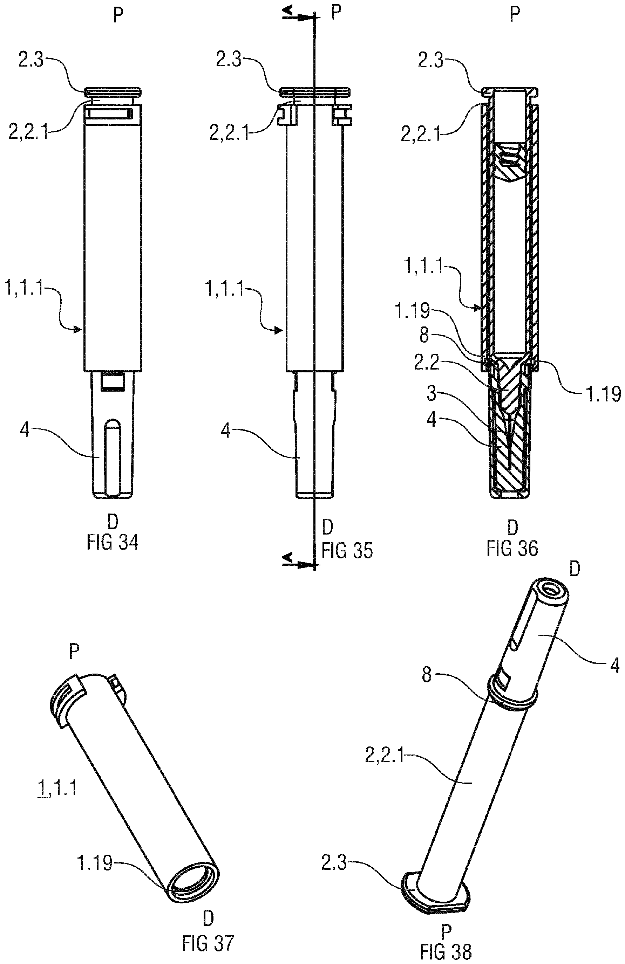

Figures 34-38 show an eighth illustrative embodiment of a syringe carrier 1. Figure 34 is a top view of an eighth embodiment of a syringe carrier 1 for supporting a syringe 2. Figure 35 is a lateral view of the syringe carrier 1 of figure 34. Figure 36 is a longitudinal section of the syringe carrier 1 of figure 34 in the section plane A-A. Figure 37 is a perspective view of the syringe carrier of figure 34 without the syringe 2. Figure 38 is another perspective view of the syringe carrier of figure 34.

As shown in Figures 34-38, the syringe carrier 1 comprises an elongate body 1.1 arranged to receive the barrel 2.1. In this exemplary embodiment, the body 1.1 has a cylindrical shape with an annular groove 1.19 adjacent its distal end which is adapted to engage a circlip 8. The circlip 8 may engage the circumferential gap between the barrel 1.2 and the RNS 4.

The syringe 2, with RNS 4 attached to the needle 3 and the circlip 8 attached to the syringe 2, may be loaded into the syringe carrier 1 by sliding the syringe 2 into the syringe carrier 1 in the distal direction D. In a non-deflected position, an outer diameter of the circlip 8 may be substantially equal to a diameter of the body 1.1. Thus, when the syringe 2 with the circlip 8 is inserted into the syringe carrier 1, the circlip 8 may deflect radially until the circlip 8 reaches the annular groove 1.19. The circlip 8 may then expand to the non-deflected position and retain the syringe 2 in an axial position relative to the syringe carrier 1. That is, the circlip 8 may engage the annular groove 1.19 and the circumferential gap between the barrel 2.1 and the RNS 4. Thus, the syringe 2 is prevented from moving axially relative to the syringe carrier 1.

In an exemplary embodiment, the proximal end may include a retainer element which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, a viewing window may be arranged in the body 1.1 for allowing visual access to the barrel 2.1 of the syringe 2 when the syringe 2 is in the syringe carrier 2.

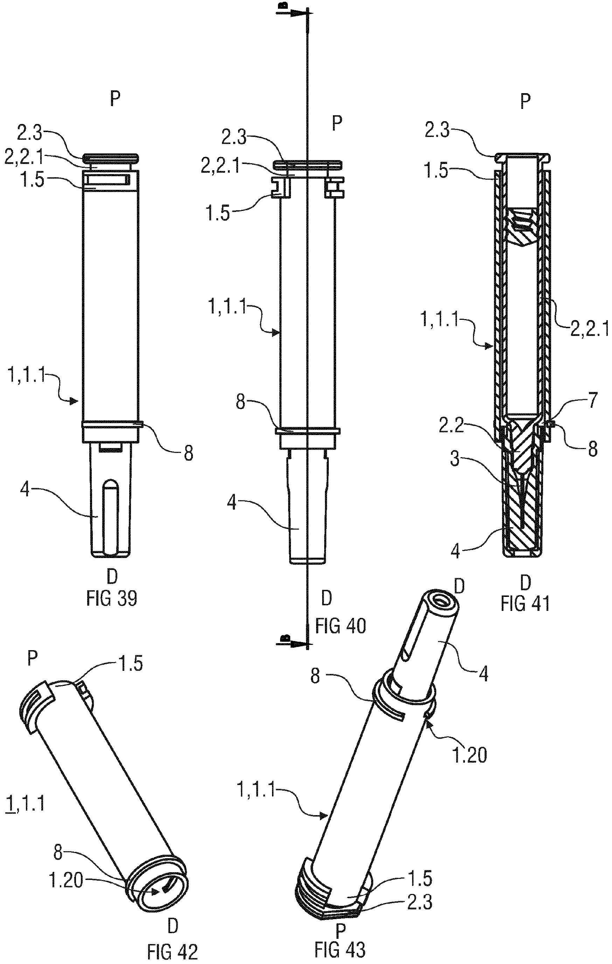

Figures 39-43 show a ninth illustrative embodiment of a syringe carrier 1. Figure 39 is a top view of a ninth embodiment of a syringe carrier 1 for supporting a syringe 2. Figure 40 is a lateral view of the syringe carrier 1 of figure 39. Figure 41 is a longitudinal section of the syringe carrier 1 of figure 39 in the section plane A-A. Figure 42 is a perspective view of the syringe carrier of figure 39 without the syringe 2. Figure 43 is another perspective view of the syringe carrier of figure 39.

As shown in Figures 39-43, the syringe carrier 1 comprises an elongate body 1.1 arranged to receive the barrel 2.1. In this exemplary embodiment, the body 1.1 has a cylindrical shape with an annular groove 1.19 having at least one aperture 1.20 adjacent its distal end which is adapted to engage a circlip 8.

The syringe 2, with RNS 4 attached to the needle 3, may be loaded into the syringe carrier 1 by sliding the syringe 2 into the syringe carrier 1 in the distal direction D. When the circumferential gap between the barrel 2.1 and the RNS 4 is aligned with the annular groove 1.19, the circlip 8 may be coupled to the body 1.1 and engage the apertures 1.20. By extending inwardly through the apertures, the circlip 8 may be coupled to the outside of the body 1.1 but engage the circumferential gap between the barrel 2.1 and the RNS 4. The engagement between the circlip 8 and the apertures 1.20 prevents the circlip 8 from translating relative to the body 1.1, and the engagement between the circlip 8 and the circumferential gap prevents the syringe 2 from moving axially relative to the syringe carrier 1.

In an exemplary embodiment, the proximal end may include a retainer element which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, a viewing window may be arranged in the body 1.1 for allowing visual access to the barrel 2.1 of the syringe 2 when the syringe 2 is in the syringe carrier 2.

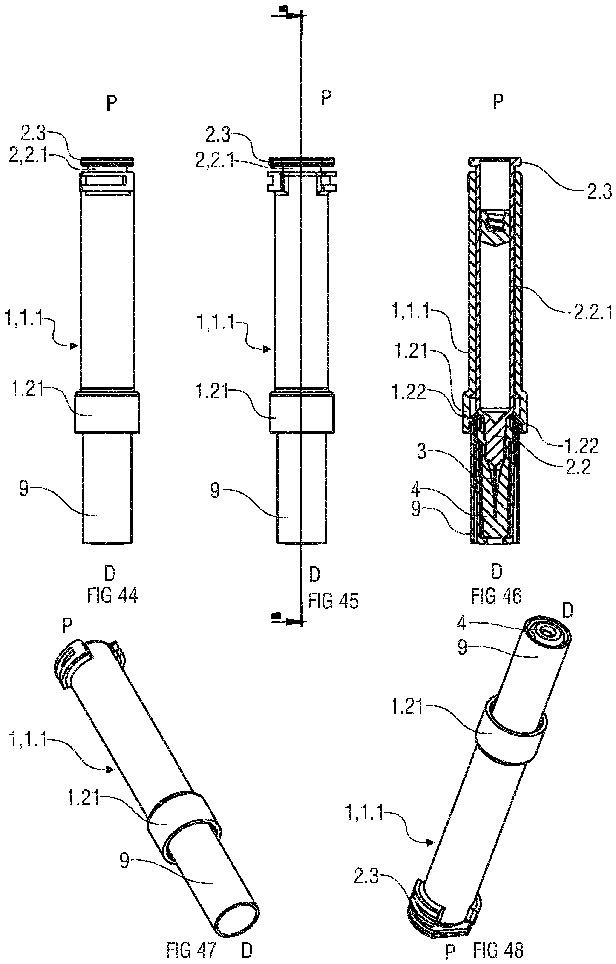

Figures 44-48 show a tenth illustrative embodiment of a syringe carrier 1 and a tool 9 for inserting a syringe 2 into the syringe carrier 1.

As shown in Figures 39-43, the syringe carrier 1 comprises an elongate body 1.1 arranged to receive the barrel 2.1. In this exemplary embodiment, the body 1.1 has an enlarged portion 1.21 on its distal end. The body 1.1 has cylindrical shape with a first diameter and the enlarged portion 1.21 has a second diameter, larger than the first diameter. The enlarged portion 1.21 has one or more resilient barbs 1.22 extending toward a longitudinal axis of the body 1.1 and angled toward a proximal end of the body 1.1.

The syringe 2, with RNS 4 attached to the needle 3, may be loaded into the syringe carrier 1 by inserting the tool 9 into the enlarged portion 1.21 of the syringe carrier 1. The tool 9 may be a cylinder having an open end adapted to receive the RNS 4. The tool 9 may have a third diameter substantially equal to the second diameter. As the tool 9 is inserted into the enlarged portion 1.21, the tool 9 engages and deflects the resilient barbs 1.22. When the barbs 1.22 are deflected, the RNS 4 can pass the barbs 1.22 in the distal direction D and extend from a distal opening of the body 1.1. When a finger flange 2.3 of the syringe 2 abuts a proximal end of the body 1.1, the tool 9 may be removed and the barbs 1.22 may engage the circumferential gap between the barrel 2.1 and the RNS 4 to prevent the syringe 2 from moving axially relative to the syringe carrier 1.

In an exemplary embodiment, the proximal end may include a retainer element which is adapted to provide an abutment surface to prevent the syringe 2 from disengaging the syringe carrier 1 in the proximal direction D.

In an exemplary embodiment, the barbs 1.22 may include proximally-facing contoured surfaces to accommodate a proximal portion of the neck 2.2 of the syringe 2 and distally-facing planar surfaces to abut the RNS 4.

In an exemplary embodiment, a viewing window may be arranged in the body 1.1 for allowing visual access to the barrel 2.1 of the syringe 2 when the syringe 2 is in the syringe carrier 2.

It is apparent to those skilled in the art that the number of deflectable arms 1.3, shoulder sections 1.4, clips 8 may be varied without departing from the scope of the invention. Likewise, all the illustrated embodiments may be implemented with or without viewing windows 5, projections 1.6, restraining features retainer elements 1.7 and clips. Different kinds of clips may likewise be applied.

Those of skill in the art will understand that modifications (additions and/or removals) of various components of the apparatuses, methods and/or systems and embodiments described herein may be made without departing from the full scope of the present invention as defined in the appended claims, which encompass such modifications and any and all equivalents thereof.

## Claims

1. A syringe carrier (1) comprising:
a body (1.1) adapted to receive a barrel (2.1) of a syringe (2), the body (1.1) including at least two sections (1.1.1) having distal ends with shoulder sections (1.4), wherein the sections (1.1.1) are coupled via at least one hinge (1.9) and are movable between an open position and a closed position, wherein the shoulder sections (1.4) are adapted to engage a circumferential gap between the barrel (2.1) of the syringe (2) and a needle shield (4) covering a needle (3) of the syringe (2) when the sections (1.1.1) are closed, wherein the syringe (2), with the needle shield (4) attached, is allowed to be loaded into the syringe carrier (1) by opening the sections (1.1.1) about the hinge and placing the syringe (2) in the syringe carrier (1), wherein the hinge (1.9) is a side hinge.

2. The syringe carrier (1) according to claim 1, wherein a first section includes a pin (1.11) adapted to engage a hole (1.10) on a second section to secure the sections (1.1.1) in the closed position.

3. The syringe carrier (1) according to any one of the preceding claims, wherein the shoulder sections (1.4) include proximally-facing contoured surfaces to accommodate a proximal portion of a neck (2.2) of the syringe (2) and distally-facing planar surfaces to abut the needle shield (4).

4. The syringe carrier (1) according to any one of the preceding claims, wherein the body (1.1) includes one or more viewing windows (5).

5. The syringe carrier (1) according to claim 4, wherein the viewing windows (5) are formed when cut-outs in the sections (1.1.1) are substantially contiguous when the sections (1.1.1) are in the closed position.

6. The syringe carrier (1) according to claim 5, wherein a projection (1.6) is formed around each cut-out, and when the sections (1.1.1) are in the non-deflected position, the projections (1.6) form an outline for the viewing window (5).

7. The syringe carrier (1) according to any one of the preceding claims, wherein the body (1.1) includes a retainer element (1.7) adapted to provide an abutment surface to prevent the syringe (2) from disengaging the syringe carrier (1) in a proximal direction (P).

8. The syringe carrier (1) according to any one of the preceding claims, wherein the two resilient sections (1.1.1), when together, have a cylindrical shape with an internal diameter corresponding to the diameter of the barrel (2.1).

9. The syringe carrier (1) according to any one of the preceding claims, wherein the shoulder sections (1.4) are shaped as portions of a circle arranged in a transverse plane with respect to a longitudinal axis of the syringe carrier (1).

10. Medicament delivery device comprising a syringe carrier (1) according to any one of the preceding claims.

11. Medicament delivery device according to claim 10, further comprising a syringe (2) having a barrel (2.1) and a needle shield (4) covering a needle (3) of the syringe (2), wherein a circumferential gap is provided between the barrel (2.1) of the syringe (2) and the needle shield (4), wherein the barrel (2.1) is received in the body (1.1), wherein the shoulder sections (1.4) engage the circumferential gap.

12. Medicament delivery device according to claim 11, wherein the syringe (2) is pre-filled with a medicament.

13. Method for assembling a syringe (2) having a barrel (2.1) and a needle shield (4) covering a needle (3) of the syringe (2) into a syringe carrier (1) according to any one of claims 2 to 12, the method comprising: opening the sections (1.1.1) about the hinge (1.9) and placing the syringe (2) in the syringe carrier (1), closing the sections (1.1.1) such that proximal shoulder sections (1.4) form circular shoulders adapted to proximally abut a finger flange (2.3) on the syringe (2), wherein closing the sections (1.1.1) furthermore causes the pin (1.11) to engage the hole (1.10) and distal shoulder sections (1.4) to distally engage the circumferential gap to prevent the syringe (2) from moving in the distal direction (D) relative to the syringe carrier (1).

## Patentansprüche

1. Spritzenträger (1), aufweisend:
einen Körper (1.1), der zur Aufnahme eines Zylinders (2.1) einer Spritze (2) ausgeführt ist, wobei der Körper (1.1) zumindest zwei Abschnitte (1.1.1) aufweist, die distale Enden mit Schulterabschnitten (1.4) haben, wobei die Abschnitte (1.1.1) über zumindest ein Scharnier (1.9) gekoppelt und zwischen einer offenen Position und einer geschlossenen Position beweglich sind, wobei die Schulterabschnitte (1.4) dazu ausgeführt sind, einen Umfangsspalt zwischen dem Zylinder (2.1) der Spritze (2) und einem Nadelschutz (4), der eine Nadel (3) der Spritze (2) abdeckt, wenn die Abschnitte (1.1.1) geschlossen sind, in Eingriff zu nehmen, wobei die Spritze (2) mit angebrachtem Nadelschutz (4) in den Spritzenträger (1) geladen werden kann, indem die Abschnitte (1.1.1) um das Scharnier geöffnet werden und die Spritze (2) in den Spritzenträger (1) platziert wird, wobei das Scharnier (1.9) ein seitliches Scharnier ist.

2. Spritzenträger (1) nach Anspruch 1, wobei ein erster Abschnitt einen Stift (1.11) aufweist, der dazu ausgeführt ist, ein Loch (1.10) an einem zweiten Abschnitt in Eingriff zu nehmen, um die Abschnitte (1.1.1) in der geschlossenen Position zu fixieren.

3. Spritzenträger (1) nach einem der vorhergehenden Ansprüche, wobei die Schulterabschnitte (1.4) proximal gerichtete, konturierte Flächen zur Aufnahme eines proximalen Abschnitts eines Halses (2.2) der Spritze (2) und distal gerichtete, planare Flächen zum Anliegen an dem Nadelschutz (4) aufweisen.

4. Spritzenträger (1) nach einem der vorhergehenden Ansprüche, wobei der Körper (1.1) ein oder mehrere Sichtfenster (5) aufweist.

5. Spritzenträger (1) nach Anspruch 4, wobei die Sichtfenster (5) ausgebildet werden, wenn Ausschnitte in den Abschnitten (1.1.1) im Wesentlichen benachbart sind, wenn die Abschnitte (1.1.1) in der geschlossenen Position sind.

6. Spritzenträger (1) nach Anspruch 5, wobei um jeden Ausschnitt ein Vorsprung (1.6) ausgebildet ist und die Vorsprünge (1.6) einen Umriss für das Sichtfenster (5) bilden, wenn die Abschnitte (1.1.1) in der nicht abgelenkten Position sind.

7. Spritzenträger (1) nach einem der vorhergehenden Ansprüche, wobei der Körper (1.1) ein Halteelement (1.7) aufweist, das zur Bereitstellung einer Anlagefläche ausgeführt ist, um zu verhindern, dass sich die Spritze (2) in einer proximalen Richtung (P) aus dem Spritzenträger (1) löst.

8. Spritzenträger (1) nach einem der vorhergehenden Ansprüche, wobei die beiden federnden Abschnitte (1.1.1), wenn sie zusammen sind, eine zylindrische Form mit einem Innendurchmesser haben, der dem Durchmesser des Zylinders (2.1) entspricht.

9. Spritzenträger (1) nach einem der vorhergehenden Ansprüche, wobei die Schulterabschnitte (1.4) als Abschnitte eines Kreises geformt sind, die in einer Querebene bezüglich einer Längsachse des Spritzenträgers (1) angeordnet sind.

10. Medikamenten-Verabreichungsvorrichtung, aufweisend einen Spritzenträger (1) nach einem der vorhergehenden Ansprüche.

11. Medikamenten-Verabreichungsvorrichtung nach Anspruch 10, ferner aufweisend eine Spritze (2) mit einem Zylinder (2.1) und einem eine Nadel (3) der Spritze (2) abdeckenden Nadelschutz (4), wobei ein Umfangsspalt zwischen dem Zylinder (2.1) der Spritze (2) und dem Nadelschutz (4) vorgesehen ist, wobei der Zylinder (2.1) in dem Körper (1.1) aufgenommen ist, wobei die Schulterabschnitte (1.4) den Umfangsspalt in Eingriff nehmen.

12. Medikamenten-Verabreichungsvorrichtung nach Anspruch 11, wobei die Spritze (2) mit einem Medikament vorgefüllt ist.

13. Verfahren zum Montieren einer Spritze (2) mit einem Zylinder (2.1) und einem Nadelschutz (4), der eine Nadel (3) der Spritze (2) abdeckt, in einem Spritzenträger (1) nach einem der Ansprüche 2 bis 12, wobei das Verfahren Folgendes aufweist: Öffnen der Abschnitte (1.1.1) um das Scharnier (1.9) und Platzieren der Spritze (2) in den Spritzenträger (1), Schließen der Abschnitte (1.1.1), so dass proximale Schulterabschnitte (1.4) kreisförmige Schultern bilden, die dazu ausgeführt sind, proximal an einem Fingerflansch (2.3) an der Spritze (2) anzuliegen, wobei durch Schließen der Abschnitte (1.1.1) ferner veranlasst wird, dass der Stift (1.11) das Loch (1.10) und distale Schulterabschnitte (1.4) in Eingriff nimmt, um den Umfangsspalt distal in Eingriff zu nehmen, um zu verhindern, dass sich die Spritze (2) bezüglich des Spritzenträgers (1) in die distale Richtung (D) bewegt.

## Revendications

1. Support (1) de seringue comprenant :
un corps (1.1) conçu pour recevoir un cylindre (2.1) d'une seringue (2), le corps (1.1) comprenant au moins deux sections (1.1.1) comportant des extrémités distales avec des sections formant épaulements (1.4), les sections (1.1.1) étant reliées par le biais d'au moins une charnière (1.9) et étant mobiles entre une position ouverte et une position fermée, les sections formant épaulements (1.4) étant conçues pour entrer en prise avec un espace circonférentiel entre le cylindre (2.1) de la seringue (2) et un élément de protection d'aiguille (4) recouvrant une aiguille (3) de la seringue (2) lorsque les sections (1.1.1) sont fermées, dans lequel la seringue (2), avec l'élément de protection d'aiguille (4) attaché, peut être chargée dans le support (1) de seringue en ouvrant les sections (1.1.1) autour de la charnière et en plaçant la seringue (2) dans le support (1) de seringue, dans lequel la charnière (1.9) est une charnière latérale.

2. Support (1) de seringue selon la revendication 1, dans lequel une première section comprend une goupille (1.11) conçue pour entrer en prise avec un trou (1.10) sur une seconde section afin d'assujettir les sections (1.1.1) dans la position fermée.

3. Support (1) de seringue selon l'une quelconque des revendications précédentes, dans lequel les sections formant épaulements (1.4) comprennent des surfaces profilées orientées dans le sens proximal destinées à accueillir une partie proximale d'un col (2.2) de la seringue (2) et des surfaces planes orientées dans le sens distal destinées à buter contre l'élément de protection d'aiguille (4).

4. Support (1) de seringue selon l'une quelconque des revendications précédentes, dans lequel le corps (1.1) comprend une ou plusieurs fenêtres de visualisation (5).

5. Support (1) de seringue selon la revendication 4, dans lequel les fenêtres de visualisation (5) sont formées lorsque des évidements dans les sections (1.1.1) sont sensiblement contigus lorsque les sections (1.1.1) sont dans la position fermée.

6. Support (1) de seringue selon la revendication 5, dans lequel une saillie (1.6) est formée autour de chaque évidement, et lorsque les sections (1.1.1) se trouvent dans la position non fléchie, les saillies (1.6) forment un contour pour la fenêtre de visualisation (5).

7. Support (1) de seringue selon l'une quelconque des revendications précédentes, dans lequel le corps (1.1) comprend un élément de retenue (1.7) conçu pour former une surface de butée destinée à empêcher la seringue (2) de se désassembler du support (1) de seringue dans une direction proximale (P).

8. Support (1) de seringue selon l'une quelconque des revendications précédentes, dans lequel les deux sections souples (1.1.1), lorsqu'elles sont ensemble, ont une forme cylindrique avec un diamètre interne correspondant au diamètre du cylindre (2.1).

9. Support (1) de seringue selon l'une quelconque des revendications précédentes, dans lequel les sections formant épaulements (1.4) ont la forme de parties d'un cercle disposé dans un plan transversal par rapport à un axe longitudinal du support (1) de seringue.

10. Dispositif d'administration de médicament comprenant un support (1) de seringue selon l'une quelconque des revendications précédentes.

11. Dispositif d'administration de médicament selon la revendication 10, comprenant en outre une seringue (2) ayant un cylindre (2.1) et un élément de protection d'aiguille (4) recouvrant une aiguille (3) de la seringue (2), dans lequel un espace circonférentiel est disposé entre le cylindre (2.1) de la seringue (2) et l'élément de protection d'aiguille (4), dans lequel le cylindre (2.1) est reçu dans le corps (1.1), dans lequel les sections formant épaulements (1.4) entrent en prise avec l'espace circonférentiel.

12. Dispositif d'administration de médicament selon la revendication 11, dans lequel la seringue (2) est préremplie d'un médicament.

13. Procédé d'assemblage d'une seringue (2) comportant un cylindre (2.1) et un élément de protection d'aiguille (4) recouvrant une aiguille (3) de la seringue (2) dans un support (1) de seringue selon l'une quelconque des revendications 2 à 12, le procédé comprenant : l'ouverture des sections (1.1.1) autour de la charnière (1.9) et le placement de la seringue (2) dans le support (1) de seringue, la fermeture des sections (1.1.1) de sorte que des sections formant épaulements (1.4) proximales forment des épaulements circulaires conçus pour buter de façon proximale contre une bride de doigt (2.3) sur la seringue (2), dans lequel la fermeture des sections (1.1.1) amène en outre la goupille (1.11) à entrer en prise avec le trou (1.10) et des sections formant épaulements (1.4) distales à entrer en prise de façon distale avec l'espace circonférentiel afin d'empêcher la seringue (2) de se déplacer dans la direction distale (D) par rapport au support (1) de seringue.
